# EUROPÄISCHE PATENTSCHRIFT

(11) **EP 1 200 043 B1**
(45) Veröffentlichungstag und Bekanntmachung des Hinweises auf die Patenterteilung: **12.03.2003**
(21) Anmeldenummer: 00958318.8
(22) Anmeldetag: 28.07.2000
(51) Int. Cl.: A61K 7/00

(54) **SONNENSCHUTZMITTEL ENTHALTEND ALKOXYLIERTE CARBONSÄUREESTER**
SUNSCREEN AGENTS COMPRISING ALCOXYLATED CARBON ACIDS
PRODUIT DE PROTECTION SOLAIRE COMPRENANT DES ACIDES CARBONIQUES ALCOXYLIQUES

(30) Priorität: 06.08.1999 DE 19937299
(43) Veröffentlichungstag der Anmeldung: 02.05.2002
(73) Patentinhaber: Cognis Deutschland GmbH & Co. KG, 40589 Düsseldorf (DE)
(72) Erfinder: EGGERS, Anke, 40215 Düsseldorf (DE); KAWA, Rolf, 40789 Monheim (DE)
(86) Internationale Anmeldenummer: EP0007326
(87) Internationale Veröffentlichungsnummer: WO01010390

(56) Entgegenhaltungen:
- EP-A- 0 661 043
- EP-A- 0 813 861
- EP-A- 0 895 776
- WO-A-99/09943
- DE-A- 2 617 817
- DE-A- 3 824 999
- DE-C- 19 641 274
- US-A- 5 220 046
- US-A- 5 916 541
- KLOKOVA, GALINA I. ET AL.: "A composition for protecting hair against ultra-violet radiation" STN INTERNATIONAL; FILE CAPLUS, AN1993:260694, 28. Juni 1993 (1993-06-28), XP002156953 & SU 1 738 284 A 7. Juni 1992 (1992-06-07)

## Beschreibung

### Gebiet der Erfindung

Die Erfindung betrifft Sonnenschutzmittel, enthaltend Ölkörper, ausgewählte anionische Emulgatoren und UV-Lichtschutzfilter sowie die Verwendung dieser Mischungen zur Herstellung von Sonnenschutzmitteln.

### Stand der Technik

Die Pigmentierung normaler Haut führt unter dem Einfluß von Sonnenstrahlung zur Bildung von Melaninen. Dabei ruft die Bestrahlung mit langwelligem UV-A Licht die Dunkelung der in der Epidermis bereits vorhandenen Melaninkörper hervor, ohne daß schädigende Folgen zu erkennen sind, während die kurzwellige UV-B Strahlung die Bildung neuen Melanins bewirkt. Ehe das schützende Pigment jedoch gebildet werden kann, unterliegt die Haut der Einwirkung der ungefilterten Strahlung, die je nach Expositionsdauer zu Hautrötungen (Erythemen), Hautentzündungen (Sonnenbrand) oder gar Brandblasen führen kann. Die mit derartigen Hautläsionen verbundenen Belastungen des Organismus, beispielsweise im Zusammenhang mit der Ausschüttung von Histaminen, kann zusätzlich zu Kopfschmerzen, Mattigkeit, Fieber, Herz- und Kreislaufstörungen und dergleichen führen. Für den Verbraucher, der sich vor den schädlichen Aspekten der Sonneneinstrahlung schützen will, bietet der Markt eine Vielzahl von Produkten, bei denen es sich ganz überwiegend um Öle und milchige Emulsionen handelt, die neben einigen Pflegestoffen vor allem UV-Lichtschutzfilter enthalten. Übersichten hierzu finden sich beispielsweise von P. Finkel in **Parf.Kosm. 76,432 (1995)** und S. Schauder in **Parf.Kosm. 76, 490 (1995).**

DE 2 617 817 und EP 895 776 offenbaren Sonnenschutzmittel mit alkoxylierten Carbonsäuren, die sich von den erfindungsgemäß verwendeten unterscheiden.

Dennoch besteht im Markt weiterhin das Bedürfnis nach Produkten mit einem verbesserten Leistungsspektrum. Besonderes Interesse gilt dabei Zubereitungen, weiche die Einarbeitung von größeren Mengen UV-Lichtschutzfiltern erlauben, ohne daß im Laufe der Lagerung eine Phasentrennung bzw. eine Sedimentation stattfindet. Eine nach der Phaseninversionstemperaturmethode hergestellte Formulierung, wie beispielsweise in der Europäischen Patentanmeldung **EP 066144 A1** (L'Oreal) beschrieben, neigt bei der Einarbeitung von größeren Mengen Titandioxid sehr rasch zur Ausscheidung des dispergierten Feststoffes. Ein weiteres Problem besteht darin, daß viele UV-Lichtschutzfilter mit den weiteren Bestandteilen der Rezeptur in Wechselwirkung treten können, was zu einer chemischen Reaktion und ebenfalls zu einer Abnahme der Lagerbeständigkeit führt. Schließlich wünscht der Verbraucher transparente Formulierungen, die auch gegenüber sehr empfindlicher Haut eine hohe hautkosmetische Verträglichkeit aufweisen. Die komplexe Aufgabe der Erfindung hat somit darin bestanden, Sonnenschutzmittel zur Verfügung zu stellen, die sich gleichzeitig durch besondere Phasenstabilität, Lagerbeständigkeit, Transparenz und Verträglichkeit gegenüber empfindlicher Haut auszeichnen.

### Beschreibung der Erfindung

Gegenstand der Erfindung sind Sonnenschutzmittel, enthaltend
(a) Ölkörper,
(b) alkoxylierte Carbonsäureester und
(c) UV-Lichtschutzfilter.

Überraschenderweise wurde gefunden, daß Zubereitungen der geschilderten Art, vorzugsweise in Form von O/W-Mikroemulsionen, selbst bei Einarbeitung größerer Mengen UV-Lichtschutzmittel äußerst lagerbeständig sind und sich durch eine besonders hohe hautkosmetische Verträglichkeit auszeichnen. Aufgrund ihrer Feinteiligkeit können sie zudem transparent sein, was zum ästhetischen Erscheinungsbild der Produkte beiträgt. Die Erfindung schließt die Erkenntnis ein, daß besonders vorteilhafte Zubereitungen erhalten werden, wenn man als Emulgatoren Mischungen von (b1) alkoxylierten Carbonsäureester und (b2) Fettsäuremonoglycerid(ether)sulfaten, Alkylethersulfaten, Alkyloligoglucosiden und/oder Fettsäurepolyglycolestersulfaten im Gewichtsverhältnis 10 : 90 bis 90 : 10 und vorzugsweise 25 : 75 bis 75 : 25 einsetzt.

### Ölkörper

Als Ölkörper (Komponente a) kommen Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare C₆-C₂₂-Fettalkoholcarbonate, Guerbetcarbonate, Ester der Benzoesäure mit linearen und/oder verzweigten C₆-C₂₂-Alkoholen (z.B. Finsolv® TN), Dialkylether, Ringöffnungsprodukte von epoxidierten Fettsäureestern mit Polyolen, Siliconöle und/oder aliphatische bzw. naphthenische Kohlenwasserstoffe in Betracht, Als Ölkörper können ferner auch Siliconverbindungen eingesetzt werden, beispielsweise Dimethylpolysiloxane, Methylphenylpolysiloxane, cyclische Silicone sowie amino-, fettsäure-, alkohol-, polyether-, epoxy-, fluor-, alkyl- und/oder glykosidmodifizierte Siliconverbindungen, die bei Raumtemperatur sowohl flüssig als auch harzförmig vorliegen können. Die Ölkörper können in den erfindungsgemäßen Mitteln in Mengen von 1 bis 90, vorzugsweise 5 bis 75 und insbesondere 10 bis 50 Gew.-% - bezogen auf den nichtwäßrigen Anteil - enthalten sein.

### Alkoxylierte Carbonsäureester

Alkoxylierte Carbonsäureester, welche die Komponente (b) bilden, sind aus dem Stand der Technik bekannt. So sind beispielsweise derartige alkoxylierte Carbonsäureester durch Veresterung von alkoxylierten Carbonsäuren mit Alkoholen zugänglich. Bevorzugt im Sinne der vorliegenden Erfindung werden die Verbindungen jedoch durch Umsetzung von Carbonsäureestern mit Alkylenoxiden unter Verwendung von Katalysatoren hergestellt, insbesondere unter Verwendung von calciniertem Hydrotalcit gemäß der Deutschen Offenlegungsschrift **DE 3914131 A,** die Verbindungen mit einer eingeschränkten Homolgenverteilung liefern. Nach diesem Verfahren können sowohl Carbonsäureester von einwertigen Alkoholen als auch von mehrwertigen Alkoholen alkoxyliert werden. Bevorzugt gemäß der vorliegenden Erfindung werden alkoxylierte Carbonsäureester der Formel **(I)** eingesetzt,

**R**^{**1**}**CO(AlkO)**_{**n**}**OR**^{**2**} **(I)**

in der R¹CO für einen aliphatischen Acylrest mit 6 bis 30 C-Atomen, AlkO für Alkylenoxid, n für Zahlen von 1 bis 30 und R² für einen aliphatischen Alkylrest mit 1 bis 8 Kohlenstoffatomen steht. AlkO steht für die Alkylenoxide, die mit den Carbonsäureestern umgesetzt werden und umfassen Ethylenoxid, Propylenoxid und/oder Butylenoxid, vorzugsweise Ethylenoxid und/oder Propylenoxid, insbesondere Ethylenoxid alleine.
Insbesondere geeignet sind alkoxylierte Carbonsäureester der Formel **(I)**, in der R¹CO für einen linearen oder verzweigten, gesättigten oder ungesättigten Acylrest mit 6 bis 22 und insbesondere 10 bis 18 Kohlenstoffatomen, AlkO für Ethylenoxid und/oder Propylenoxid, n durchschnittlich für Zahlen 5 bis 20 und R² für einen aliphatischen Alkylrest mit 1 bis 8, vorzugsweise 1 bis 4 Kohlenstoffatomen und insbesondere Methyl steht.
Bevorzugte Acylreste leiten sich von Carbonsäuren mit 6 bis 22 Kohlenstoffatomen natürlicher oder synthetischer Herkunft ab, insbesondere von linearen, gesättigten und/oder ungesättigten Fettsäuren einschließlich technischer Gemische derselben, wie sie durch Fettspaltung aus tierischen und/oder pflanzlichen Fetten und Ölen zugänglich sind, zum Beispiel aus Kokosöl, Palmkernöl, Palmöl, Sojaöl, Sonnenblumenöl, Rüböl, Baumwollsaatöl, Fischöl, Rindertalg und Schweineschmalz. Beispiele für derartige Carbonsäuren sind Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und/oder Erucasäure.
Insbesondere geeignet sind alkoxylierte Carbonsäureester der Formel **(I)**, in der R¹CO für einen linearen oder verzweigten, aliphatischen, gesättigten und/oder ungesättigten Acylrest mit 10 bis 18 Kohlenstoffatomen, AlkO für Ethylenoxid und/oder Propylenoxid, vorzugsweise Ethylenoxid, n für Zahlen von 5 bis 20 und R² für einen Methylrest steht. Beispiele für derartige Verbindungen sind mit im Durchschnitt 5, 7, 9 oder 11 Mol Ethylenoxid alkoxylierte Laurinsäuremethylester, Kokosfettsäuremethylester und Talgfettsäuremethylester.

### Monoglycerid(ether)sulfate

Monoglyceridsulfate und Monoglyceridethersulfate, die als anionische Co-Emulgatoren in Betracht kommen, stellen bekannte anionische Tenside dar, die nach den einschlägigen Methoden der präparativen organischen Chemie erhalten werden können. Üblicherweise geht man zu ihrer Herstellung von Triglyceriden aus, die gegebenenfalls nach Ethoxylierung zu den Monoglyceriden umgeestert und nachfolgend sulfatiert und neutralisiert werden. Gleichfalls ist es möglich, die Partialglyceride mit geeigneten Sulfatierungsmitteln, vorzugsweise gasförmiges Schwefeltrioxid oder Chlorsulfonsäure umzusetzen [vgl. **EP 0561825 B1, EP 0561999 B1** (Henkel)]. Die neutralisierten Stoffe können - falls gewünscht - einer Ultrafiltration unterworfen werden, um den Elektrolytgehalt auf ein gewünschtes Maß zu vermindern **[DE 4204700 A1** (Henkel)]. Übersichten zur Chemie der Monoglyceridsulfate sind beispielsweise von A.K. Biswas et al. in **J.Am.Oil.Chem.Soc. 37, 171 (1960)** und F.U. Ahmed **J.Am.Oil.Chem. Soc. 67, 8 (1990)** erschienen. Die im Sinne der Erfindung einzusetzenden Monoglycerid(ether)sulfate folgen vorzugsweise der Formel **(II)**, 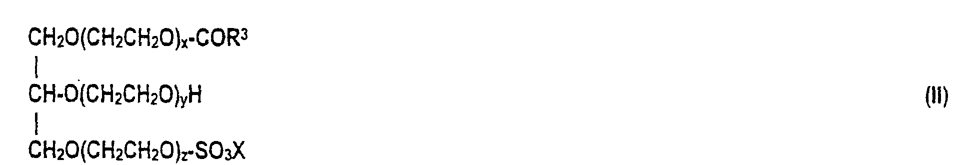 in der R³CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel **(II)** eingesetzt, in der R³CO für einen linearen Acylrest mit 8 bis 18 Kohlenstoffatomen steht.

### Alkylethersulfate

Alkylethersulfate ("Ethersulfate"), die ebenfalls als anionische Co-Emulgatoren in Betracht kommen, stellen bekannte Tenside dar, die großtechnisch durch SO₃- oder Chlorsulfonsäure (CSA)-Sulfatierung von Fettalkohol- oder Oxoalkoholpolyglycolethern und nachfolgende Neutralisation hergestellt werden. Im Sinne der Erfindung kommen Ethersulfate in Betracht, die der Formel **(III)** folgen,

**R**^{**4**}**O-(CH**_{**2**}**CH**_{**2**}**O)**_{**m**}**SO**_{**3**}**X (III)**

in der R⁴ für einen linearen oder verzweigten Alkyl- und/oder Alkenylrest mit 6 bis 22 Kohlenstoffatomen, m für Zahlen von 1 bis 10 und X für ein Alkali- und/oder Erdalkalimetall, Ammonium, Alkylammonium, Alkanolammonium oder Glucammonium steht. Typische Beispiele sind die Sulfate von Anlagerungsprodukten von durchschnittlich 1 bis 10 und insbesondere 2 bis 5 Mol Ethylenoxid an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoteylalkohol, Behenylalkohol, Erucylalkohol und Brassidylalkohol sowie deren technische Mischungen, in Form ihrer Natrium- und/oder Magnesiumsatze. Die Ethersulfate können dabei sowohl eine konventionelle als auch eine eingeengte Homologenverteilung aufweisen. Besonders bevorzugt ist der Einsatz von Ethersulfaten auf Basis von Addukten von durchschnittlich 2 bis 3 Mol Ethylenoxid an technische C_{12/14}- bzw. C_{12/18}- Kokosfettalkoholfraktionen in Form ihrer Natrium- und/oder Magnesiumsalze.

### Alkyl- und/oder Alkenyloligoglykoside

Alkyl- und Alkenyloligoglykoside, die als nichtionische Co-Emulgatoren in Betracht kommen, stellen bekannte Tenside dar, die der Formel **(IV)** folgen,

**R**^{**5**}**O-[G]**_{**p**} **(IV)**

in der R⁵ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die Schriften **EP 0301298 A1** und **WO 90/03977** verwiesen. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligoglucoside. Die Indexzahl p in der allgemeinen Formel **(IV)** gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R⁵ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R⁵ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

### Fettsäurepolyglycolestersulfate

Fettsäurepolyglycolestersulfate, die als nichtionische Co-Emulgatoren in Betracht kommen, stellen bekannte Tenside dar, die der Formel **(V)** folgen,

**R**^{**6**}**COO(AO)**_{**q**}**SO**_{**3**}**X (V)**

in der R⁶CO für einen linearen oder verzweigten, gesättigten oder ungesättigten Acylrest mit 6 bis 22 Kohlenstoffatomen, q für Zahlen von durchschnittlich 1 bis 3 und AO für einen CH₂CH₂O-, CH₂CH(CH₃)O- und/oder CH(CH₃)CH₂O-Rest und X für ein Alkali- und/oder Erdalkalimetall, Ammonium, Alkylammonium, Alkanolammonium oder Glucammonium steht.
Sie werden durch Sulfatierung der entsprechenden Fettsäurepolyglycolester hergestellt. Diese wiederum sind nach den einschlägigen präparativen Verfahren der organischen Chemie erhältlich. Hierzu wird Ethylenoxid, Propylenoxid oder deren Gemisch in random oder Blockverteilung an die entsprechenden Fettsäuren angelagert, wobei diese Reaktion säurekatalysiert, vorzugsweise aber in Gegenwart von Basen, wie z.B. Natriummethylat oder calciniertem Hydrotalcit erfolgt. Wird ein Alkoxylierungsgrad von 1 gewünscht, können die Zwischenprodukte auch durch Veresterung der Fettsäuren mit einem entsprechenden Alkylenglycol hergestellt werden. Die Sulfatierung der Fettsäurepolyglycolester kann in an sich bekannter Weise mit Chlorsulfonsäure, Amidosulfonsäure oder vorzugsweise gasförmigem Schwefeltrioxid durchgeführt werden, wobei das molare Einsatzverhältnis zwischen Fettsäurepolyglycolester und Sulfatierungsmittel im Bereich von 1 : 0,95 bis 1 : 1,2, vorzugsweise 1 : 1 bis 1 : 1,1 und die Reaktionstemperatur 30 bis 80 und vorzugsweise 50 bis 60°C betragen kann. Es ist ferner möglich, die Fettsäurepolyglycolester zu untersulfatieren, d.h. deutlich weniger Sulfatierungsmittel einzusetzen, als dies für eine vollständige Umsetzung stöchiometrisch erforderlich wäre. Wählt man beispielsweise molare Einsatzmengen von Fettsäurepolyglycolester zu Sulfatierungsmittel von 1 : 0,5 bis 1 : 0,95 werden Mischungen von Fettsäurepolyglycolestersulfaten und Fettsäurepolyglycolestern erhalten, die für eine ganze Reihe von Anwendungen ebenfalls vorteilhaft sind. Um eine Hydrolyse zu vermeiden ist es dabei sehr wichtig, die Neutralisation bei einem pH-Wert im Bereich von 5 bis 9, vorzugsweise 7 bis 8 durchzuführen. Typische Beispiele für geeignete Ausgangsstoffe sind die Anlagerungsprodukte von 1 bis 3 Mol Ethylenoxid und/oder Propylenoxid, vorzugsweise aber die Addukte mit 1 Mol Ethylenoxid oder 1 Mol Propylenoxid an Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die dann wie oben beschrieben sulfatiert und neutralisiert werden. Vorzugsweise werden Fettsäurepolyglycolestersulfate der Formel **(V)** eingesetzt, in der R⁶CO für einen Acylrest mit 12 bis 14 bzw. 12 bis 18 Kohlenstoffatomen, q für durchschnittlich 1 oder 2, AO für eine CH₂CH₂O-Gruppe und X für Natrium oder Ammonium steht, wie beispielsweise Laurinsäure+1EO-sulfat-Natriumsalz, Laurinsäure+1EO-sulfat-Ammoniumsalz, Kokosfettsäure+1EO-sulfat-Natriumsalz, Kokosfettsäure+1EO-sulfat-Ammoniumsalz, Talgfettsäure+1EO-sulfat-Natriumsalz, Talgfettsäure+1EO-sulfat-Ammoniumsalz sowie deren Mischungen.

Der Anteil der genannten Co-Emulgatoren liegt in Summe üblicherweise - bezogen auf den nichtwäßrigen Teil - bei 5 bis 75, vorzugsweise 10 bis 50 und insbesondere 15 bis 30 Gew.-%.

### UV-Lichtschutzfilter

Unter **UV-Lichtschutzfaktoren** sind beispielsweise bei Raumtemperatur flüssig oder kristallin vorliegende organische Substanzen (Lichtschutzfilter, Komponente a) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
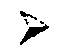 3-Benzylidencampher bzw. 3-Benzylidennorcampher und dessen Derivate, z.B. 3-(4-Methylbenzyliden)campher wie in der **EP 0693471 B1** beschrieben;
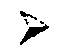 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
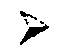 Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
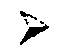 Ester der Salicylsäure, vorzugsweise Salicylsäure-2-ethylhexylester, Salicylsäure-4-isopropylbenzylester, Salicylsäurehomomenthylester;
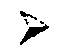 Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
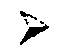 Ester der Benzalmalonsäure, vorzugsweise 4-Methoxybenzmalonsäuredi-2-ethylhexylester;
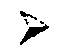 Triazinderivate, wie z.B. 2,4,6-Trianilino-(p-carbo-2'-ethyl-1'-hexyloxy)-1,3,5-triazin und Octyl Triazon, wie in der **EP 0818450 A1** beschrieben oder Dioctyl Butamido Triazone (Uvasorb® HEB);
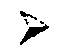 Propan-1,3-dione, wie z.B. 1-(4-tert.Butylphenyl)-3-(4'methoxyphenyl)propan-1,3-dion;
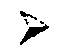 Ketotricyclo(5.2.1.0)decan-Derivate, wie in der **EP 0694521 B1** beschrieben.

Als wasserlösliche Substanzen kommen in Frage:
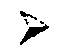 2-Phenylbenzimidazol-5-sulfonsäure und deren Alkali-, Erdalkali-, Ammonium-, Alkylammonium-, Alkanolammonium- und Glucammoniumsalze;
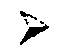 Sulfonsäurederivate von Benzophenonen, vorzugsweise 2-Hydroxy-4-methoxybenzophenon-5-sulfonsäure und ihre Salze;
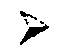 Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen, wie beschrieben in der **DE 19712033 A1** (BASF). Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P. Finkel in **SÖFW-Journal 122, 543 (1996)** und **Parfümerie und Kosmetik 3 (1999)** zu entnehmen.

### Mikroemulsionen

In einer bevorzugten Ausführungsform der Erfindung liegen die Sonnenschutzmittel in Form von Mikroemulsionen, insbesondere O/W-Mikroemulsionen vor. Mikroemulsionen stellen optisch isotrope, thermodynamisch stabile Systeme dar, die Ölkomponenten, Emulgatoren und Wasser enthalten. Das klare bzw. transparente Aussehen der Mikroemulsionen ist eine Folge der geringen Teilchengröße der dispergierten Emulsionströpfchen, die im wesentlichen unter 300, vorzugsweise bei 50 bis 300 nm liegen. Im Bereich zwischen 100 und 300 nm werden dabei feinteilige, in der Durchsicht braunrote und im Auflicht bläulich schimmernde Mikroemulsionen erhalten. Im Bereich unterhalb eines Tröpfchendurchmessers von 100 nm sind Mikroemulsionen klar. Die Herstellung der Mittel erfolgt vorzugsweise in einem Kaltverfahren, bei dem man Ölkörper und Emulgatoren mit Wasser emulgiert und anschließend die UV-Lichtschutzfilter sowie gegebenenfalls weitere Zusatzstoffe hinzugibt. Durch Zusatz von Wasser und/oder Hydrotropen kann der gewünschte Aktivsubstanzgehalt eingestellt werden.

### Gewerbliche Anwendbarkeit

Die erfindungsgemäßen Mittel zeichnen sich durch eine hohe Transparenz und Phasenstabilität bei besonders vorteilhafter hautkosmetischer Verträglichkeit aus. Typische Zubereitungen haben die folgende Zusammensetzung:
(a) 1 bis 90, vorzugsweise 5 bis 80 Gew.-% Ölkörper,
(b) 0,05 bis 90, vorzugsweise 0,2 bis 10 Gew.-% Emulgatoren und
(c) 1 bis 90, vorzugsweise 5 bis 75 Gew.-% UV-Lichtschutzfilter,
mit der Maßgabe, daß sich die Mengenangaben zu 100 Gew.-% ergänzen. Innerhalb der genannten Konzentrationsbereiche werden besonders stabile und feinteilige Mikroemulsionen erhalten.

Ein weiterer Gegenstand der Erfindung betrifft schließlich die Verwendung von O/W-Mikroemulsionen, enthaltend
(a) Ölkörper,
(b) alkoxylierte Carbonsäureester und
(c) UV-Lichtschutzfilter.
zur Herstellung von Sonnenschutzmitteln.

Als weitere Inhaltsstoffe können sie in untergeordneten Mengen weitere, mit den anderen Inhaltsstoffen kompatible Aniontenside enthalten. Typische Beispiele sind Seifen, Alkylbenzolsulfonate, Alkansulfonate, Olefinsulfonate, Alkylethersulfonate, Glycerinethersulfonate, α-Methylestersulfonate, Sulfofettsäuren, Glycerinethersulfate, Fettsäureethersulfate, Hydroxymischethersulfate, Fettsäure-amid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acyl-glutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Die erfindungsgemäßen Mittel können ferner als weitere Hilfs- und Zusatzstoffe Co-Emulgatoren, Überfettungsmittel, Stabilisatoren, Wachse, Konsistenzgeber, Verdickungsmittel, Kationpolymere, biogene Wirkstoffe, Konservierungsmittel, Hydrotrope, Solubilisatoren, Farb- und Duftstoffe enthalten. Als **Co-Emulgatoren** kommen beispielsweise nichtionogene Tenside aus mindestens einer der folgenden Gruppen in Frage:
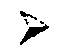 Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
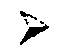 Alkyl- und/oder Alkenyloligoglykoside mit 8 bis 22 Kohlenstoffatomen im Alk(en)ylrest und deren ethoxylierte Analoga;
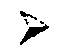 Anlagerungsprodukte von 1 bis 15 Mol Ethylenoxid an Ricinusöl und/oder gehärtetes Ricinusöl;
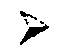 Anlagerungsprodukte von 15 bis 60 Mol Ethylenoxid an Ricinusöl und/oder gehärtetes Ricinusöl;
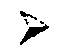 Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
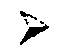 Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
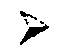 Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin.
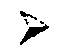 Mono-, Di- und Trialkylphosphate sowie Mono-, Di- und/oder Tri-PEG-alkylphosphate und deren Salze;
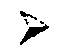 Wollwachsalkohole;
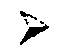 Polysiloxan-Polyalkyl-Polyether-Copolymere bzw. entsprechende Derivate;
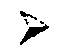 Polyalkylenglycole sowie
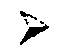 Glycerincarbonat.

Die **Anlagerungsprodukte von Ethylenoxid und/oder von Propylenoxid** an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 2024051 PS** als Rückfettungsmittel für kosmetische Zubereitungen bekannt.

Typische Beispiele für geeignete **Partialglyceride** sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

Als **Sorbitanester** kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

Typische Beispiele für geeignete **Polyglycerinester** sind Polyglyceryl-2 Dipolyhydroxystearate (Dehymuls® PGPH), Polyglycerin-3-Diisostearate (Lameform® TGl), Polyglyceryl-4 Isostearate (Isolan® Gl 34), Polyglyceryl-3 Oleate (z. B. Cremophor 60 32, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische.

Beispiele für weitere geeignete **Polyolester** sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

Weiterhin können als Emulgatoren **zwitterionische Tenside** verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytiscne Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin.

Schließiich kommen auch **Kationtenside** als Emulgatoren in Betracht, wobei solche vom Typ der Esterquats, vorzugsweise methylquaternierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohfenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Konsistenzgeber** kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten.

Geeignete **Verdickungsmittel** sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsatz und Ammoniumchlorid.

Geeignete **kationische Polymere** sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed collagen (Lamequat®L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR 2252840 A** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/Acrylat-Copolymere, Octylacrylamid/Methylmethacrylat/tert.Butylamin-oethylmethacrylat/2-Hydroxyproyl-methacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cyclische Silicone sowie amino-, fettsäure-, Alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil. 91, 27 (1976).**

Typische Beispiele für **Fette** sind Glyceride, wie beispielsweise Triglyceride auf Basis von Fettsäuren mit 6 bis 22 Kohlenstoffatomen, Hydroxystearinsäure, Isostearinsäure, Ölsäure, Ricinolsäure, Linolsäure, Linolensäure, Erucasäure, Weinsäure, Citronensäure, Äpfelsäure sowie deren technische Gemische, die untergeordnet aus dem Herstellungsprozeß noch geringe Mengen an Triglycerid enthalten können, als **Wachse** kommen u.a. natürliche Wachse, wie z.B. Candelillawachs, Carnaubawachs, Japanwachs, Espartograswachs, Korkwachs, Guarumawachs, Reis-keimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie **Lecithine** und **Phospholipide** in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC) bezeichnet und folgen der allgemeinen Formel 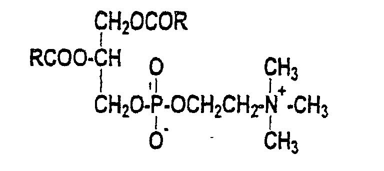 wobei R typischerweise für lineare aliphatische Kohlenwasserstoffreste mit 15 bis 17 Kohlenstoffatomen und bis zu 4 cis-Doppelbindungen steht. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

Als **Stabilisatoren** können Metallsalze von Fettsäuren, wie z.B. Magnesium-, Aluminium- und/oder Zinkstearat bzw. -ricinoleat eingesetzt werden. Ferner kommen die entsprechenden Alkali- und/oder Erdalkalisalze bzw. deren Mischungen in Betracht.

Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.

Kosmetische **Deodorantien** (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

Als **keimhemmende Mittel** sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 24,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonolaurat (GML), Diglycerinmonocaprinat (DMC), Salicylsäure-N-alkylamide wie z. B. Salicylsäure-n-octylamid oder Salicylsäure-n-decylamid.

Als **Enzyminhibitoren** sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, Henkel KGaA, Düsseldorf/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat.

Als **Geruchsabsorber** eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicytat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

**Antitranspirantien** (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
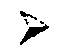 adstringierende Wirkstoffe,
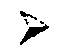 Ölkomponenten,
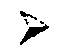 nichtionische Emulgatoren,
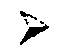 Coemulgatoren,
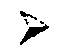 Konsistenzgeber,
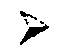 Hilfsstoffe wie z. B. Verdicker oder Komplexierungsmittel und/oder
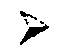 nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin.
Daneben können in Antitranspirantien übliche öllösliche und wasserlösliche Hilfsmittel in geringeren Mengen enthalten sein. Solche öllöslichen Hilfsmittel können z.B. sein:
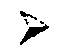 entzündungshemmende, hautschützende oder wohlriechende ätherische Öle,
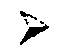 synthetische hautschützende Wirkstoffe und/oder
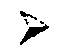 öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

Als **Antischuppenmittel** können Octopirox® (1-Hydroxy-4-methyl-6-(2,4,4-trimythylpentyl)-2-(1H)-pyridon-monoethanolaminsalz), Baypival, Pirocton Olamin, Ketoconazol®, (4-Acetyl-1-{4-[2-(2.4-dichlorphenyl)r-2-(1H-imidazol-1-ylmethyl)-1,3-dioxylan-c-4-ylmethoxyphenyl}piperazin, Selendisulfid, Schwefel kolloidal, Schwefelpolyehtylenglykolsorbitanmonooleat, Schwefelrizinolpolyehtoxylat, Schwefelteer Destillate, Salicylsäure (bzw. in Kombination mit Hexachlorophen), Undexylensäure Monoethanolamid Sulfosuccinat Na-Salz, Lamepon® UD (Protein-Undecylensäurekondensat, Zinkpyrethion, Aluminiumpyrition und Magnesiumpyrithion / Dipyrithion-Magnesiomsulfat eingesetzt werden.

Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

Als **Quellmittel** für wäßrige Phasen können Montmorillonite, Clay Mineralstoffe, Pemulen sowie alkyl-modifizierte Carbopoltypen (Goodrich) dienen. Weitere geeignete Polymere bzw. Quellmittel können der Übersicht von R.Lochhead in **Cosm.Toil. 108, 95 (1993)** entnommen werden.

Neben den beiden vorgenannten Gruppen primärer Lichtschutzstoffe können auch sekundäre Lichtschutzmittel vom Typ der **Antioxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-Apalmitat) sowie Koniferylbenzoat des Benzoeharzes, Rutinsäure und deren Derivate, α-Glycosylrutin, Ferulasäure, Furfurylidenglucitol, Carnosin, Butylhydroxytotuol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Zur Verbesserung des Fließverhaltens können ferner **Hydrotrope,** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
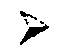 Glycerin;
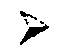 Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
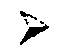 technische Oligoglyceringemische mit einem Eigenkondensationsgrad von 1,5 bis 10 wie etwa technische Diglyceringemische mit einem Diglyceringehalt von 40 bis 50 Gew.-%;
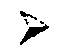 Methyolverbindungen, wie insbesondere Trimethylolethan, Trimethylolpropan, Trimethylolbutan, Pentaerythrit und Dipentaerythrit;
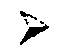 Niedrigalkylglucoside, insbesondere solche mit 1 bis 8 Kohlenstoffen im Alkylrest, wie beispielsweise Methyl- und Butylglucosid;
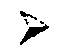 Zuckeralkohole mit 5 bis 12 Kohlenstoffatomen, wie beispielsweise Sorbit oder Mannit,
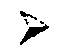 Zucker mit 5 bis 12 Kohlenstoffatomen, wie beispielsweise Glucose oder Saccharose;
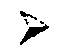 Aminozucker, wie beispielsweise Glucamin;
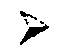 Dialkoholamine, wie Diethanolamin oder 2-Amino-1,3-propandiol.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als **Insekten-Repellentien** kommen N,N-Diethyl-m-toluamid, 1,2-Pentandiol oder Ethyl Butylacetylaminopropionate in Frage, als **Selbstbräuner** eignet sich Dihydroxyaceton, Als **Tyrosinhinbitoren,** die die Bildung von Melanin verhindern und Anwendung in Depigmentierungsmitteln finden, kommen beispielsweise Arbutin, Kojisäure, Cumarinsäure und Ascorbinsäure (Vitamin C) in Frage.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### Beispiele

Die erfindungsgemäßen O/W-Mikroemulsionen 1 bis 6 wurden in einem Kaltverfahren hergestellt. Dazu wurden die Ölkörper zusammen mit den öllöslichen bzw. öldispergierbaren UV-Lichtschutzfiltern und den Emulgatoren in Wasser emulgiert; wachsartige Komponenten wurden aufgeschmolzen und bei leicht erhöhter Temperatur eingearbeitet, wasserlösliche bzw. wasserdispergierbare UV-Filter zusammen mit der wäßrigen Phase eingebracht. Es wurden wasserklare Mikroemulsionen erhalten. Die Transmission der Emulsionen wurde photometrisch bei 650 nm bestimmt. Die Beurteilung der Stabilität erfolgte nach einer Lagerung von 12 Wochen bei 40 °C. Hierbei bedeuten (+++) = keine Phasentrennung/Sedimentation und (++) keine Phasentrennung/geringfügige Trübung. Die Ergebnisse sind in Tabelle 1 zusammengefaßt.

**Tabelle 1**

| **O/W-Mikroemulsionen** | | | | | | |
|---|---|---|---|---|---|---|
| **Komponente /Performance** | **1** | **2** | **3** | **4** | **5** | **6** |
| C_{12/14} Fettsäure + 7 EO-methylester | 12,0 | 6,0 | 4,0 | 6,0 | 6,0 | 3,0 |
| Sodium Coco Monoglycerol Sulfate | - | 6,0 | - | - | 2,0 | - |
| Sodium Laureth Sulfate | - | - | 2,0 | 3,0 | - | 4,0 |
| Dodecyl Polyglucose | | - | 6,0 | 3,0 | 4,0 | 5,0 |
| Glyceryl Oleate | 6,0 | - | - | - | - | - |
| Octyldodecanol | 3,0 | 15,0 | - | - | - | 15,0 |
| Dicaprylyl Ether | - | 15,0 | - | - | - | 20,0 |
| Decyl Oleate | - | - | 35,0 | - | - | - |
| Caprylic/Capric Triglyceride | - | - | - | 35,0 | - | - |
| Cetearyl Isononanoate | - | - | - | - | 35,0 | - |
| Octyl Methoxycinnamate | 5,0 | 3,0 | - | 4,0 | 5,0 | - |
| 4-Methylbenzyliden Camphor | 1,0 | - | 2,0 | 1,0 | - | 2,0 |
| Octyl Triazone | - | 1,0 | 2,0 | 2,0 | - | 1,5 |
| Octocrylene | | 2,0 | 4,0 | 3,0 | | |
| Phenylbenzimidazole Sulfonic Acid (Sodium Salt) | 2,0 | 1,0 | | | | 1,5 |
| Butyl Methoxydibenzoylmethane | 1,0 | 1,5 | 1,5 | | | 1,5 |
| Zinc Oxide (micronisiert) | | | | 6,0 | 2,0 | |
| Titanium Dioxide (oberflächenbehandelt) | - | - | - | - | 5,0 | - |
| Tocopherol | - | - | - | - | - | 10,0 |
| Wasser | ad 100 | | | | | |
| ***Lagerstabilität*** | +++ | ++ | ++ | +++ | ++ | +++ |
| ***Transmission*** **[%]** | 82 | 89 | 92 | 90 | 96 | 99 |

## Patentansprüche

1. Sonnenschutzmittel, enthaltend
(a) Ölkörper,
(b) alkoxylierte Carbonsäureester der Formel **(I)**,
**R**^{**1**}**CO(AlkO)**_{**n**}**OR**^{**2**} **(I)**
in der R¹CO für einen aliphatischen Acylrest mit 6 bis 30 C-Atomen, AlkO für Alkylenoxid, n für Zahlen von 1 bis 30 und R² für einen aliphatischen Alkylrest mit 1 bis 8 Kohlenstoffatomen steht und
(c) UV-Lichtschutzfilter
mit der Maßgabe, dass die alkoxylierten Carbonsäureester (b1) in Kombination mit (b2) Fettsäuremonoglycerid(ether)sulfaten, Alkylethersulfaten, Alkyloligoglucosiden und/oder Fettsäurepolyglycolestersulfaten im Gewichtsverhältnis 10 : 90 bis 90 : 10 einsetzt werden.

2. Mittel nach Anspruch 1, **dadurch gekennzeichnet, dass** sie als Ölkörper enthalten, die ausgewählt sind aus der Gruppe, die gebildet wird von Guerbetalkoholen auf Basis von Fettalkoholen mit 6 bis 18, Estern von linearen C₆-C₂₂-Fettsäuren mit linearen C₆-C₂₂-Fettalkoholen, Estern von verzweigten C₆-C₁₃-Carbonsäuren mit linearen C₆-C₂₂-Fettalkoholen, Estern von linearen C₆-C₂₂-Fettsäuren mit verzweigten Alkoholen, Estern von linearen und/oder verzweigten Fettsäuren mit mehrwertigen Alkoholen und/oder Guerbetalkoholen, Triglyceriden auf Basis C₆-C₁₀-Fettsäuren, flüssigen Mono-/Di-/Triglyceridmischungen auf Basis von C₆-C₁₈-Fettsäuren, Estern von C₆-C₂₂-Fettalkoholen und/oder Guerbetalkoholen mit aromatischen Carbonsäuren, Estern von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Ölen, verzweigten primären Alkoholen, substituierten Cyclohexanen, linearen C₆-C₂₂-Fettalkoholcarbonaten, Guerbetcarbonaten, Estern der Benzoesäure mit linearen und/oder verzweigten C₆-C₂₂-Alkoholen, Dialkylethern, Ringöffnungsprodukten von epoxidierten Fettsäureestern mit Polyolen, aliphatischen bzw. naphthenischen Kohlenwasserstoffen sowie Siliconölen.

3. Mittel nach mindestens einem der Ansprüche 1 und/oder 2, **dadurch gekennzeichnet, dass** sie als Emulgatorkomponente alkoxylierte Carbonsäureester der Formel **(I)** enthalten, in der R¹CO für einen linearen oder verzweigten, gesättigten oder ungesättigten Acylrest mit 10 bis 18 Kohlenstoffatomen, AlkO für Ethylenoxid und/oder Propylenoxid, n für Zahlen von 5 bis 20 und R² für Methyl steht.

4. Mittel nach mindestens einem der Ansprüche 1 bis 3, **dadurch gekennzeichnet, dass** sie als Emulgatorkomponente weiterhin Monoglycerid(ether)sulfate, Alkylethersulfate, Alkyl- und Alkenyloligoglykoside und/oder Fettsäurepolyglycolestersulfate enthalten.

5. Mittel nach mindestens einem der Ansprüche 1 bis 4, **dadurch gekennzeichnet, dass** sie UV-Lichtschutzfilter enthalten, die ausgewählt sind aus der Gruppe, die gebildet wird von 3-Benzylidencampher und dessen Derivaten, 4-Aminobenzoesäurederivaten, Estern der Zimtsäure, Estern der Salicylsäure, Derivaten des Benzophenons, Estern der Benzalmalonsäure, Triazinderivaten, Propan-1,3-dionen, 2-Phenylbenzimidazol-5-sulfonsäure und deren Salzen, Sulfonsäurederivaten von Benzophenonen, Sulfonsäurederivaten des 3-Benzylidencamphers, sowie Benzoylmethanderivaten.

6. Mittel nach den Ansprüchen 1 bis 5, **dadurch gekennzeichnet, dass** sie als UV-Lichtschutzfilter feindisperse Metalloxide bzw. Salze enthalten, die ausgewählt sind aus der Gruppe, die gebildet wird von Titandioxid, Zinkoxid, Eisenoxid, Aluminiumoxid, Ceroxid, Zirkoniumoxid, Silicate (Talk) und Bariumsulfat.

7. Mittel nach den Ansprüchen 1 bis 6, **dadurch gekennzeichnet, dass** sie als UV-Lichtschutzmittel Antioxidantien enthalten, die ausgewählt sind aus der Gruppe, die gebildet wird von Superoxid-Dismutase, Tocopherolen (Vitamin E) und Ascorbinsäure (Vitamin C).

8. Verwendung von O/W-Mikroemulsionen, enthaltend
(a) Ölkörper,
(b) alkoxylierte Carbonsäureester der Formel **(I)**,
**R**^{**1**}**CO(AlkO)**_{**n**}**OR**^{**2**} **(I)**
in der R¹CO für einen aliphatischen Acylrest mit 6 bis 30 C-Atomen, AlkO für Alkylenoxid, n für Zahlen von 1 bis 30 und R² für einen aliphatischen Alkylrest mit 1 bis 8 Kohlenstoffatomen steht und
c) UV-Lichtschutzfilter
mit der Maßgabe, dass die alkoxylierten Carbonsäureester (b1) in Kombination mit (b2) Fettsäuremonoglycerid(ether)sulfaten, Alkylethersulfaten, Alkyloligoglucosiden und/oder Fettsäurepolyglycolestersulfaten im Gewichtsverhältnis 10 : 90 bis 90:10 vorliegen, zur Herstellung von Sonnenschutzmitteln.

## Claims

1. Sun protection compositions containing
(a) oil components,
(b) alkoxylated carboxylic acid esters corresponding to formula **(I)**:
**R**^{**1**}**CO(AlkO)**_{**n**}**OR**^{**2**} **(I)**
in which R¹CO is an aliphatic acyl group containing 6 to 30 carbon atoms, AlkO stands for alkylene oxide, n is a number of 1 to 30 and R² is an aliphatic alkyl group containing 1 to 8 carbon atoms,
and
(c) UV filters,
with the proviso that the alkoxylated carboxylic acid esters (b1) are used in combination with (b2) fatty acid monoglyceride (ether) sulfates, alkyl ether sulfates, alkyl oligoglucosides and/or fatty acid polyglycol ester sulfates in a ratio by weight of 10:90 to 90:10.

2. Compositions as claimed in claim 1, **characterized in that** they contain oil components selected from the group consisting of Guerbet alcohols based on C₆₋₁₈ fatty alcohols, esters of linear C₆₋₂₂ fatty acids with linear C₆₋₂₂ fatty alcohols, esters of branched C₆₋₁₃ carboxylic acids with linear C₆₋₂₂ fatty alcohols, esters of linear C₆₋₂₂ fatty acids with branched alcohols, esters of linear and/or branched fatty acids with polyhydric alcohols and/or Guerbet alcohols, triglycerides based on C₆₋₁₀ fatty acids, liquid mono-/di-/triglyceride mixtures based on C₆₋₁₈ fatty acids, esters of C₆₋₂₂ fatty alcohols and/or Guerbet alcohols with aromatic carboxylic acids, esters of C₂₋₁₂ dicarboxylic acids with linear or branched alcohols containing 1 to 22 carbon atoms or polyols containing 2 to 10 carbon atoms and 2 to 6 hydroxyl groups, vegetable oils, branched primary alcohols, substituted cyclohexanes, linear C₆₋₂₂ fatty alcohol carbonates, Guerbet carbonates, esters of benzoic acid with linear and/or branched C₆₋₂₂ alcohols, dialkyl ethers, ring opening products of epoxidized fatty acid esters with polyols, aliphatic or naphthenic hydrocarbons and silicone oils.

3. Compositions as claimed in at least one of claims 1 and/or 2, **characterized in that** they contain alkoxylated carboxylic acid esters corresponding to formula **(I)**, in which R¹CO is a linear or branched, saturated or unsaturated acyl group containing 10 to 18 carbon atoms, AlkO stands for ethylene oxide and/or propylene oxide, n is a number of 5 to 20 and R² is a methyl group,
as the emulsifier component.

4. Compositions as claimed in at least one of claims 1 to 3, **characterized in that** they additionally contain monoglyceride (ether) sulfates, alkyl ether sulfates, alkyl and alkenyl oligoglycosides and/or fatty acid polyglycol ester sulfates as emulsifier component.

5. Compositions as claimed in at least one of claims 1 to 4, **characterized in that** they contain UV filters selected from the group consisting of 3-benzylidene camphor and derivatives thereof, 4-aminobenzoic acid derivatives, esters of cinnamic acid, esters of salicylic acid, derivatives of benzophenone, esters of benzalmalonic acid, triazine derivatives, propane-1,3-diones, 2-phenylbenzimidazole-5-sulfonic acid and salts thereof, sulfonic acid derivatives of benzophenones, sulfonic acid derivatives of 3-benzylidene camphor and benzoyl methane derivatives.

6. Compositions as claimed in claims 1 to 5, **characterized in that** they contain finely dispersed metal oxides or salts selected from the group consisting of titanium dioxide, zinc oxide, iron oxide, aluminium oxide, cerium oxide, zirconium oxide, silicates (talcum) and barium sulfate as UV filters.

7. Compositions as claimed in claims 1 to 6, **characterized in that** they contain antioxidants selected from the group consisting of superoxid-dismutase, tocopherols (vitamin E) and ascorbic acid (vitamin C) as UV filters.

8. The use of o/w microemulsions containing
(a) oil components,
(b) alkoxylated carboxylic acid esters corresponding to formula **(I):**
**R**^{**1**}**CO(AlkO)**_{**n**}**OR**^{**2**} **(I)**
in which R¹CO is an aliphatic acyl group containing 6 to 30 carbon atoms, AlkO stands for alkylene oxide, n is a number of 1 to 30 and R² is an aliphatic alkyl group containing 1 to 8 carbon atoms,
and
(c) UV filters,
with the proviso that the alkoxylated carboxylic acid esters (b1) are used in combination with (b2) fatty acid monoglyceride (ether) sulfates, alkyl ether sulfates, alkyl oligoglucosides and/or fatty acid polyglycol ester sulfates in a ratio by weight of 10:90 to 90:10,
for the production of sun protection compositions.

## Revendications

1. Agent protecteur solaire contenant
a) un composé huileux,
b) un ester d'acide carboxylique alkoxylé de formule (I)
R¹CO(AlkO)ₙOR² (I)
dans laquelle F¹CO représente un reste acyle aliphatique ayant de 6 à 30 atomes de carbone, AlkO représente un oxyde d'alkylène, n représente des nombres allant de 1 à 30 et R² représente un reste alkyle aliphatique ayant de 1 à 8 atomes de carbone,
c) un filtre de protection contre la lumière UV, avec la restriction que les esters d'acide carboxylique alkoxylés (b1) sont mis en oeuvre en combinaison avec (b2) des sulfates (d'éther) de monoglycéride d'acide gras, des sulfates d'éther d'alkyle, des alkyloligoglucosides et/ou des sulfates d'ester de polyglycol d'acide gras dans un rapport en poids de 10 :90 à 90:10.

2. Composition selon la revendication 1,
**caractérisée en ce qu'**
elle renferme comme composé huileux, ceux qui sont choisis dans le groupe formé des alcools de Guerbet à base d'alcool gras ayant de 6 à 18, des esters d'acide gras linéaires en C₆-C₂₂ avec des alcools gras linéaires en C₆-C₂₂, des esters d'acide carboxylique ramifié en C₆-C₁₃ avec des alcools gras linéaires en C₆-C₂₂, des esters d'acide gras linéaires en C₆-C₂₂ avec des alcools ramifiés, des esters d'acide gras linéaire et/ou ramifié avec des alcools plurifonctionnels et/ou des alcools de Guerbet, des triglycérides à base d'acides gras en C₆-C₁₀, des mélanges liquides de mono/di/triglycérides à base d'acide gras en C₆-C₁₈, des esters d'alcools gras en C₆-C₂₂ et/ou d'alcools de Guerbet avec des acides carboxyliques aromatiques des esters d'acide dicarboxylique en C₂-C₁₂ avec des alcools linéaires ou ramifiés ayant de 1 à 22 atomes de carbone ou avec des polyols ayant de 2 à 10 atomes de carbone et de 2 à 6 groupes hydroxyle, des huiles végétales, des alcools primaires ramifiés, des cyclohéxanes substitués, des carbonates d'alcool gras linéaires en C₆-C₂₂, des carbonates de Guerbet, des esters d'acide benzoïque avec des alcools linéaires et/ou ramifiés en C₆-C₂₂, des éthers dialkyliques, des produits d'ouverture du cycle d'esters d'acide gras époxydés avec des polyols, des hydrocarbures aliphatiques ou naphténiques ainsi que des huiles de silicone.

3. Composition selon au moins une des revendications 1 et/ou 2,
**caractérisée en ce qu'**
elle renferme comme composant d'agent émulsionnant, des esters d'acide carboxylique alkoxylés de formule (I) dans laquelle R¹CO représente un reste acyle linéaire ou ramifié, saturé ou non saturé ayant de 10 à 18 atomes de carbone, AlkO représente de l'oxyde d'éthylène et/ou de l'oxyde de propylène, n représente des chiffres allant de 5 à 20, et R² représente un méthyle.

4. Composition selon au moins une des revendications 1 à 3,
**caractérisée en ce qu'**
elle renferme comme composant d'agent émulsionnant, en outre des sulfates (d'éther) de monoglycéride, des sulfates d'éther d'alkyle, des alkyl et alkényloligoglycosides et/ou des sulfates d'esters de glycol d'acide gras.

5. Composition selon au moins une des revendications 1 à 4,
**caractérisé en ce qu'**elle contient des filtres contre la lumière UV qui sont choisies dans le groupe formé du 3-benzilidène camphre et ses dérivés, des dérivés d'acide 4-aminobenzoïque, des esters d'acide cinnanique, des esters d'acide salicylique, des dérivés de la benzophénone, des esters de l'acide benzol malonique, des dérivés de la triazine, des propane-1,3-diones, de l'acide 2-phénylbenzimidazole-5-sulfonique et de ses sels, des dérivés d'acide sulfonique, benzophénones, des dérivés d'acide sulfonique du 3-benzylidène camphre ainsi que des dérivés du benzoylméthane.

6. Composition selon les revendications 1 à 5,
**caractérisé en ce qu'**
elle renferme comme filtre contre la lumière UV, des oxydes métalliques finement dispersés ou leurs sels qui sont choisis dans le groupe formé du dioxyde de titane, de l'oxyde de zinc, de l'oxyde de fer, de l'oxyde d'aluminium, de l'oxyde de cérium, de l'oxyde de zirconium, des silicates (talc) et du sulfate de baryum.

7. Composition selon les revendications 1 à 6,
**caractérisée en ce qu'**
elle contient comme agent protecteur contre la lumière UV, des antioxydants qui sont choisis dans le groupe formé de la superoxydismutase, des tocophérols (vitamine E) et de l'acide ascorbique (vitamine C).

8. Utilisation des micro-émulsions O/W huile/eau contenant
a) un composé huileux
b) des esters d'acide carboxylique alkoxylés de formule (I)
R¹CO(AlkO)ₙOR²
dans laquelle R¹CO représente un reste acyle aliphatique ayant de 6 à 30 atomes de carbone, AlkO représente un oxyde d'alkylène, n représente des nombres allant de 1 à 30, et R² représente un reste alkyle aliphatique ayant de 1 à 8 atomes de carbone et
c) un filtre contre la lumière UV, avec la restriction que les esters d'acide carboxylique alkoxylés (b1) se présentent en combinaison avec (b2) des sulfates (d'éther) de monoglycérides d'acide gras, des sulfates d'éther d'alkyle, des alkyloligoglucosides, et/ou des sulfates d'ester de polyglycol d'acide gras dans un rapport en poids allant de 10 :90 à 90 :10, en vue de la production d'agents protecteurs solaires.
